# DEMANDE DE BREVET EUROPEEN

(11) **EP 1 616 537 A1**
(43) Date de publication de la demande: **18.01.2006**
(21) Numéro de dépôt: 05368014.6
(22) Date de dépôt: 11.07.2005
(51) Int. Cl.: A61F 2/06

(54) **Prothèses vasculaires délivrant en permanence des médicaments et des produits bio-actifs divers**

(30) Priorité: 15.07.2004 FR 0407844
(71) Demandeur: Bourgogne, P.M., 06300 Nice (FR)
(72) Inventeur: Bourgogne, P.M., 06300 Nice (FR)

(57) **Abrégé**

Prothèses artificielles bio actives pour la délivrance IN SITU de médicaments directement dans les organes à traiter et pour la réparation et/on le remplacement de vaisseaux naturels, comportant un ou des réservoirs de grand volume (2) à parois élastiques (1,1bis) rechargeables avec des produits bio actifs et permettant l'introduction d'une sonde ou de tout autre instrument, une valve (14) s'oppose au reflux du sang.

Une pastille (6) micro-poreuse ou percée de trous de très faible diamètre permet la diffusion des produits actifs.

Posées en dérivation, sans pastille (6), elles comportent, outre les réservoirs (1,1bis), deux conduits concentriques, l'un externe élastique (16) et l'autre interne (17) micro-poreux ou percé de trous (19) de très faible diamètre. Les réservoirs sont reliés aux vaisseaux naturels par des pièces de raccordement (4) comportant une plaque (4bis) courbe de grande surface facilitant l'anastomose classique ou par collage. De faibles dimensions elles permettent leur mise en place par voie de chirurgie mini-invasive et endoscopique.

## Description

### Domaine technique

La présente invention concerne des prothèses artificielles destinées à délivrer IN SITU toutes sortes de produits bio-actifs et médicamenteux pour traiter, de façon optimale, de nombreuses maladies ainsi qu'à réparer ou à remplacer des vaisseaux obstrués, malades, détériorés, et en particulier les artères coronaires tout en ne présentant pas les risques habituels de sténose ou de resténose des prothèses artificielles connus en chirurgie cardiaque ou vasculaire. Ces prothèses se comportent comme des réservoirs, facilement rechargeables, de produits actifs délivrés en permanence, et si besoin pendant toute la vie des patients, dans un ou plusieurs vaisseaux naturels, irriguant par exemple un organe malade tel foie, pancréas, estomac etc etc. ou abouchés à l'amont, par exemple, d'une plaque d'athérome formant bouchon ou à l'amont d' un ressort ou " STENT " posé depuis longtemps ou au cours de la même intervention, elles peuvent être posées à coeur battant, à thorax ouvert ou par les techniques de la chirurgie mini-invasive ou endoscopique et aussi bien anastomosée à l'aide de fils et d'aiguilles ou d'agrafes que collées de façon particulièrement simple et efficace. Dans les cas où elles même et les vaisseaux auxquels elles sont reliées devraient être explorés ou débouchés ils peuvent l'être très facilement à tout moment et sans intervention chirurgicale ni anesthésie, hormis éventuellement une légère insensibilisation locale.

### Etat de la technique antérieure :

Jusqu'à présent tous les essais de prothèses artificielles vasculaires se sont heurtés au fait que, surtout pour les conduits de petit diamètre, elles se sont rapidement obstruées avec formation de plaques et de bouchons nécessitant une nouvelle intervention chirurgicale, de même les " Stents " très fréquemment mis en place pour dilater et maintenir ouverts les vaisseaux provoquent par leur présence et dans de nombreux cas des sténoses ou des resténoses.

Récemment sont apparus des stents " actifs " imprégnés de substances délivrant pendant un laps de temps court, de l'ordre de quelques semaines, un produit anti-agrégant limitant les risques de resténose, mais ces substances imprégnant superficiellement la paroi des prothèses et des stents et diffusant lentement sont en quantité trop faible pour avoir un effet durable et n'ont de ce fait qu'une efficacité limitée dans le temps.

C'est pourquoi il serait particulièrement utile que ces prothèses artificielles puissent délivrer IN SITU, à des doses minimes (pour n'avoir que des effets secondaires totalement négligeables sur le reste de l'organisme ), en PERMANENCE et si besoin durant toute la vie des patients, des produits médicamenteux pour le traitement de nombreuses maladies et/ou anti-agrégants et anti-prolifération tissulaire réduisant ainsi presque totalement tout risque de sténose, et que, encore mieux, la conformation de ces prothèses artificielles soit telle qu'il devienne facile de les explorer et de les déboucher ainsi que les vaisseaux naturels sur lesquels elles sont anastomosées, sans intervention chirurgicale ni anesthésie hormis une éventuelle insensibilisation ponctuelle.

C'est ce résultat qui est atteint grâce à la présente invention.

### Exposé de l'invention.

Toutes les dispositions qui suivent peuvent s'appliquer aussi bien aux prothèses artificielles Monotronculaires ne concernant qu'un seul vaisseau, qu' aux prothèses pluritronculaires concernant un ensemble de plusieurs vaisseaux.

L'objet de l'invention est de délivrer IN SITU des produits actes et des médicaments, permettant de traiter de nombreuses maladies avec des résultats bien supérieurs à ceux obtenus classiquement quand l' introduction de ces substances est faite directement dans la circulation générale, en effet avec ces prothèses les doses délivrées peuvent être beaucoup plus faibles avec une réduction très sensible sinon totale des effets secondaires nuisibles.

L'objet de l'invention est également de permettre le débouchage et/ou le remplacement de tout ou partie des vaisseaux obstrués, malades ou endommagés, et en particulier des coronaires, par des prothèses artificielles bio actives réalisées en matériaux biocompatibles sans risques de sténose ou de resténose.

Dans une première application de cette invention une prothèse artificielle est constituée d'une paroi, généralement de forme globalement tubulaire, faisant office de RESERVOIR PRINCIPAL, facilement rechargeable, pour des produits actifs et/ou médicamenteux et/ou anti-agrégant capables de traiter un ou des organes malades et/ou de s'opposer à la prolifération de tissus indésirables et à la formation de plaques et de bouchons susceptibles de ralentir ou d'interrompre la circulation sanguine, ces produits actifs sont instillés en permanence pendant de très longues périodes, et si besoin pendant toute la vie du patient directement in situ dans les zones à traiter, une pièce de raccordement assure la liaison entre le "Réservoir Principal " et le ou les vaisseaux naturels récepteurs ou avec un ou des " Stents " mis en place dans le ou les vaisseaux défectueux, soit au cours de la même intervention, soit plusieurs mois ou plusieurs années auparavant.

Une autre application essentielle consiste à utiliser ces prothèses artificielles " en DERIVATION " pour REMPLACER une ou des parties de vaisseaux trop détériorées pour être encore fonctionnelles, dans ce cas la partie aval du "Réservoir Principal " est prolongée par une double paroi globalement tubulure anastomosée en dérivation sur le ou les vaisseaux naturels défaillants par l'intermédiaire de pièces de raccordement, l'espace libre entre ces deux parois concentriques fait office de " réservoir complémentaire " permettant un stockage supplémentaire de produit actif. La partie Aval de ces parois concentriques peut être avantageusement prolongée par un deuxième "Réservoir Principal" similaire et symétrique à celui relié à leur extrémité Amont, le volume de l'ensemble de ces réservoirs est suffisant pour n'avoir besoin d'être rechargé qu'à intervalles très éloignés, par exemple tous les trois ou six mois ou plus et ceci aussi longtemps que nécessaire, si besoin pendant toute la vie du patient.

Dans les deux cas l'anastomose entre prothèses artificielles et vaisseaux naturels se fait, grâce à des Pièces de Raccordement, par les techniques classiques avec fil et aiguille ou par agrafes, mais elle peut, encore mieux et plus facilement, se faire par collage, il sera avantageux d'anastomoser ces prothèses artificielles aux vaisseaux récepteurs, et en particulier en cas de collage, AVANT de percer ces vaisseaux au droit de l'anastomose ceci permettant au chirurgien de travailler en présence d'un minimum de sang.

Un autre avantage tout à fait déterminant de l'invention est de supprimer la nécessité de prélever sur le patient un ou des vaisseaux naturels sains et de dimensions convenables comme cela est le cas dans les techniques classiques qui, devant l'impossibilité d'utiliser une prothèse artificielle pour diverses raisons et en particulier, à cause des risques élevés de sténose, imposent le prélèvement de vaisseaux naturels tels que des artères, mammaire interne, radiale, gastroépiploique, ou de veines telle la veine saphène (il s'agit le plus souvent de l'artère mammaire interne dans le cas des pontages ), ce qui est une opération importante et comportant des risques car l'ensemble de cette opération, y compris le pontage, se fait le plus souvent sous anesthésie générale à coeur arrêté et sous circulation extracorporelle avec tous les risques inhérents.

Les prothèses artificielles objet de la présente invention peuvent être mises en place en de nombreux points du corps humain et en particulier à thorax ouvert mais, bien mieux, leur faible diamètre permet leur introduction et leur mise en place par les méthodes de la chirurgie mini-invasive et/ou endoscopique, à coeur battant et sans nécessiter une circulation extra corporelle.

Les produits actifs sont utilisés à très faible concentration et diffusent très lentement soit au travers d'une pastille micro-poreuse amovible placée à l'extrémité aval du "Réservoir Principal" ou dans le cas de prothèse " En Dérivation au travers de la paroi du conduit interne qui est en contact direct avec le sang, cette paroi est micro-poreuse on percée de trous de très faible diamètre compris entre quelques dizaines de nanomètres et quelques dizaines de micromètres, cette diffusion se produisant sur la plus grande partie de la longueur de cette paroi, aucune agrégation ou prolifération néfaste susceptible de freiner la circulation du sang n'est donc possible puisque cette diffusion intéresse la quasi totalité de la surface interne de la prothèse en contact avec le sang et qu'elle s'effectue en permanence 24 h/24 et 365 jours par an.

La quantité de produit actif diffusée peut être modulée à volonté en fonction du taux de dilution, de la porosité, du diamètre des trous, de leur nombre et de la pression repant dans le ou les réservoirs.

Certains des différents produits actifs utilisables sont susceptibles, selon la quantité journalière diffusée et entrant donc dans la circulation sanguine, de provoquer des effets secondaires indésirables, la RAPAMYCINE (SIROLIMUS) par exemple est un anti-agrégant mais aussi un immunosuppresseur susceptible d'augmenter le taux de cholestérol et des triglycérides, il est donc important de contrôler la quantité journalière délivrée de ces produits actifs pour qu'ils restent en dessous du seuil de nocivité, ceci est facile puisque lors de la conception de la prothèse il est possible de jouer sur les paramètres cités ci avant qui règlent les quantités journalières diffusées, de plus ces produits étant délivrés IN SITU il suffit de quantités infimes pour obtenir le résultat désiré sans pour autant générer d'effets secondaires gênants.

Pour pouvoir traverser la Pastille amovible ou la paroi interne de la prothèse il doit régner dans le ou les réservoirs une pression légèrement supérieure à celle de la pression sanguine, c'est pourquoi la paroi externe de la prothèse en dérivation et celle du ou des "Réservoirs Principaux "la prolongeant est prévue dilatable et élastique, les remplissages de l'ensemble prothèse/réservoirs se font donc sous une légère pression qui diminue lentement au fur et à mesure de la vidange par diffusion afin de rester toujours légèrement plus forte que le pression sanguine moyenne jusqu'au prochain remplissage. L'élasticité de ces parois est la source de la force qui pousse le liquide actif au travers des Pastilles micro-poreuses amovibles ou de la paroi interne de la prothèse en dérivation, cette élasticité est elle même réglable en fonction de la nature du matériau biocompatible constituant les parois externes, par exemple un silicone, un latex anallergique, un polyuréthane, un polymère etc.. et de leur épaisseur.

Dans le cas de la prothèse installée en dérivation et pour éviter que la paroi interne ne s'écrase sous l'effet de cette pression il est prévu d'incorporer un ressort dans cette paroi lors de la fabrication de la prothèse, ce ressort, genre ressort à boudin, pouvant être avantageusement en acier inoxydable.

Une autre caractéristique essentielle de l'invention consiste en ce que les " Réservoirs Principaux " de ces prothèses artificielles peuvent être, entre de nombreuses autres possibilités, reliés au sternum du patient.

La ou les deux extrémités amont du ou des " Réservoirs Principaux ", coté sternum, peuvent être simplement glissées sous la peau ou être scellées dans un trou percé dans le sternum auquel elle sont alors fermement liées par collage, ossification périphérique ou tout autre moyen, elles pourraient bien entendu tout aussi bien être apparentes à la surface de la peau.

Ceci permet de remplir périodiquement les " Réservoirs Principaux " de produit actif à l'aide d'une seringue à contrôle de pression munie d'une aiguille hypodermique traversant la peau et ouvrant une vanne de préférence élastique empêchant le reflux du sang et du liquide actif. Par mesure de sécurité supplémentaire il est possible de compléter ce dispositif par une vis ou un bouchon d'étanchéité.

Ces mêmes extrémités des " Réservoirs Principaux " peuvent également servir à l'introduction éventuelle d'une sonde équipée de fibres optiques, d'une mini caméra, d'un ballonnet gonflable, d' un appareil de curetage capable de supprimer d'éventuels bouchons dans la prothèse ou dans les vaisseaux naturels récepteurs en aval et/ou en amont de la prothèse ou de tous autres instruments. Ces interventions, parfaitement bénignes peuvent s'effectuer en quelques minutes quand nécessaire, sans avoir à créer quelque ouverture corporelle que ce soit hormis le trou de quelques millimètres de diamètre permettant le passage de l'aiguille ou de la sonde et ceci avec éventuellement une simple insensibilisation locale.

Comme indiqué ci dessus on peut prévoir un ou deux "Réservoirs Principaux " de produit actif : s'il y en a un seul il sera relié au vaisseau naturel à l'amont de la zone à Traiter et/ou défaillante et/ou du Stent, de cette façon il sera possible d'intervenir non seulement sur la zone à traiter et/ou défaillante et/ou sténosée mais aussi sur le Stent dans le cas ou il y en a un, ainsi que sur tout le vaisseau naturel et l'organe situés à l'aval de l'anastomose, s'il y a deux "Réservoirs Principaux" ils seront reliés l'un à l'aval et l'autre à l'amont de la dérivation permettant ainsi d'intervenir sur la totalité de la prothèse et des vaisseaux et/ou organes concernés.

Une autre caractéristique essentielle de l'invention est que l'anastomose entre vaisseaux naturels et prothèses artificielles peut se faire soit de façon classique par suture avec fils ou agrafes mais encore beaucoup mieux par collage. En effet dans une anastomose classique en " T ", cas le plus classique, on n'a comme surface de contact entre la prothèse rapportée en dérivation et le vaisseau récepteur qu' une surface égale à celle de la section droite de la prothèse rapportée, or cette surface est extrêmement faible et la paroi du vaisseau récepteur est fréquemment n mauvais état ce qui rend l'anastomose par fil très délicate et fragile et elle manque parfois d'etancheite. L'anastomose entre deux prothèses artificielles peut être réalisée de la même façon.

Dans la configuration de l'invention la Pièce de Raccordement de la prothèse artificielle reliée au vaisseau récepteur a une surface beaucoup plus imposante, de plus elle est courbe et épouse donc parfaitement la paroi externe du vaisseau récepteur, le collage peut donc être réalisé par simple contact, à coeur battant et même sur des vaisseaux en très mauvais état, et sur une surface très importante les difficultés d'une suture par fil ou agrafes ce qui est capital et bien entendu encore plus dans les pontages par endoscopie sans ouverture large du thorax.

Il existe plusieurs colles biologiques employées depuis longtemps qui ont fait leurs preuves en particulier dans le traitement des anévrismes, et depuis peu sont apparues des colles cyano-acrylates purifiées à prise rapide ( en quelques secondes) qui facilitent énormément l'anastomose et diminuent dans de très importantes proportions le temps nécessaire à sa réalisation tout en augmentant sensiblement la qualité de la liaison.

La solidité des liaisons entre vaisseaux récepteurs et prothèses artificielles peut également être renforcée en incorporant à la surface des zones de la prothèse en contact avec le vaisseau naturel une couche d'un produit facilitant la prolifération tissulaire, il en est de même pour la liaison entre l'extrémité de la paroi du ou des "Réservoirs Principaux" au sternum, par exemple de la poudre de corail.

Il est également possible de percer des trous de faible diamètre à la périphérie de la pièce de raccordement destinés à être comblés par les cellules proliférantes et à assurer ainsi une étanchéité et un ancrage parfaits

Sur le plan pratique l'ensemble " Réservoirs principaux ", Dérivation, " Pièces de Raccordement " peut être fabriqué en une seule ou en plusieurs pièces, par tous les procèdes classiques et également grâce à la méthode bien connue de la " Cire perdue " et par la technique de fabrication des gants en élastomères.

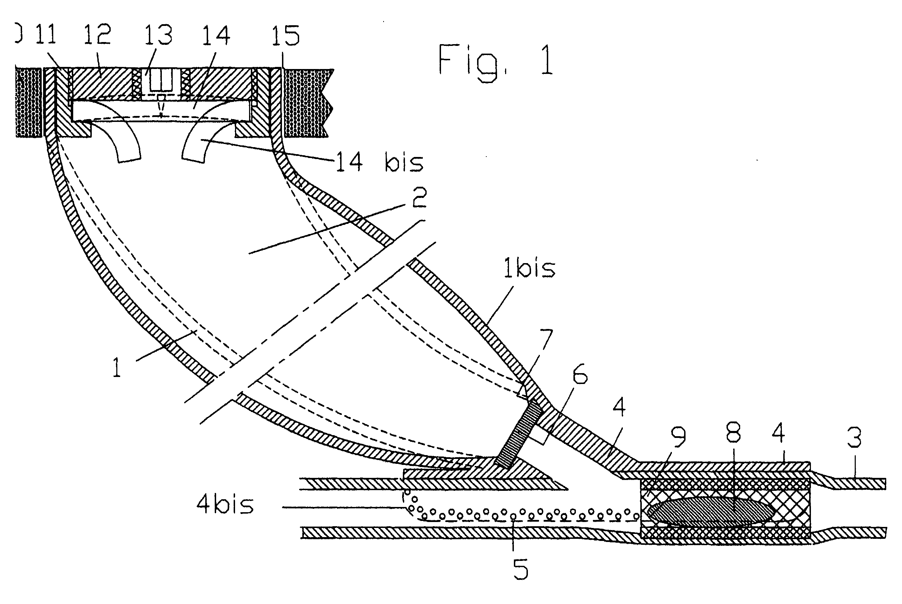

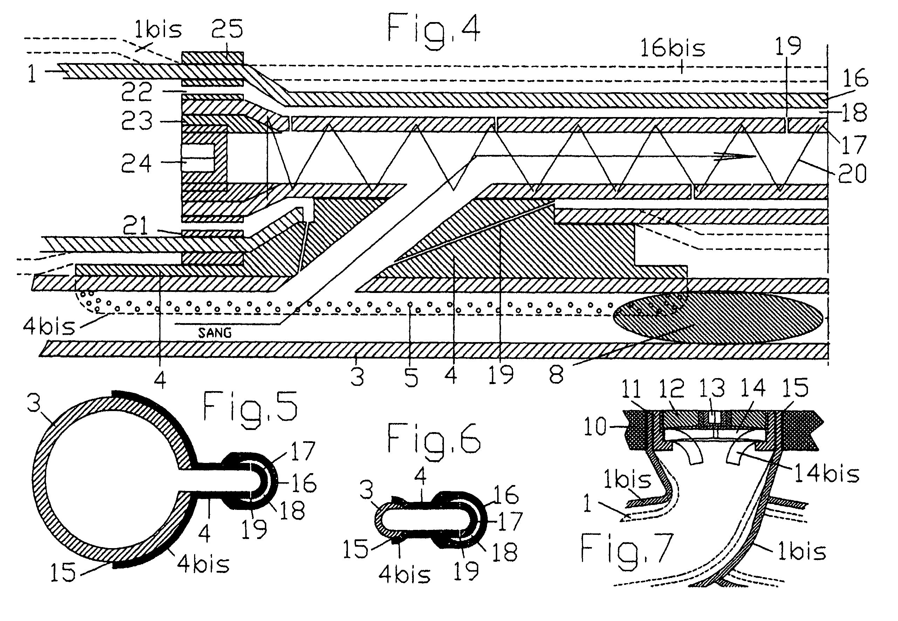

La description détaillée des moyens mis en oeuvre par l'invention est illustrée à l'aide des figures 1 à 7
La fig. 1 est une coupe sur un "Réservoir principal " avec son raccordement au vaisseau naturel
La Fig.2 est une vue d'ensemble d'une prothèse monotronculaire " En Dérivation "avec deux" Réservoirs Principaux"
La Fig.3 est une vue d'ensemble d'une prothèse pluritronculaire"
La fig. 4 est une vue de détail agrandie du raccordement entre une prothèse " En Dérivation " et un vaisseau naturel
La Fig. 5 est une coupe perpendiculaire à l'axe principal d'une prothèse " En dérivation " sur un gros vaisseau type aorte.
La Fig. 6 est une coupe perpendiculaire à l'axe principal d'une prothèse " En dérivation " sur un petit vaisseau type coronaire
La Fig. 7 est une coupe sur le raccordement d'un " Réservoir Principal " au sternum.

La prothèse vasculaire artificielle active objet de la présente invention est constituée essentiellement d'un réservoir dont les parois élastiques (1) en position dégonflée et (1 bis) en position de remplissage maximum délimitent un volume (2) rempli d'un produit biologiquement actif, il est relié à un organe et/ou à un ou des vaisseaux naturels récepteur (3) par une ou des pièces de raccordement (4) dont la partie inférieure (4 bis) est courbée selon un rayon proche de celui du vaisseau ou de l'organe récepteurs, cette partie (4 bis) peut être percée de petits trous (5) destinés à être comblés par les cellules proliférantes émises par le vaisseau naturel afin d'assurer une liaison solide et étanche entre prothèse et vaisseau. Une Pastille micro-poreuse ou percée de nombreux trous de faible diamètre (6) permet une diffusion lente contrôlée du produit actif en direction du vaisseau naturel et/ou de l'organe à traiter, elle est maintenue en place par un bourrelet élastique déformable (7).

L'anastomose entre prothèse et vaisseau naturel peut se faire par les moyens classiques, fil, aiguille, agrafes, mais encore mieux par collage entre la partie courbe (4 bis) de la pièce de raccordement (4) et le vaisseau (3), avec éventuellement utilisation de produits (15) favorisant l'adhérence. La surface de contact entre la plaque courbe (4 bis) et le vaisseau (3) est très importante et plusieurs fois supérieure à celle de la section droite des vaisseaux utilisés dans les pontages classiques, cette grande surface ajoutée à l'intimité du contact entre plaque (4 bis ) et vaisseau (3) permet soit une suture classique très performante soit encore mieux un collage facile, rapide et particulièrement efficace à l'aide de colles biocompatibles connues

Le percement du vaisseau naturel peut se faire avantageusement après la mise en place de la prothèse artificielle et le bouchon ( d'athérome par exemple ) (8) peut alors être éliminé grâce à l'introduction d'une sonde adaptée à cet usage introduite après enlèvement de la vis ou du bouchon (13). Si un " STENT " (9) avait été mis en place auparavant il peut être ainsi débouché ou extrait et éventuellement remplacé, ou bien un ou des " STENTS " neufs peuvent être mis en place.

L'extrémité Amont de ce Réservoir Principal ( 1,1bis,2) pourra être simplement glissé sous la peau en de nombreux endroits du corps d'accès facile, ou, par exemple, fixée au sternum (10) après avoir percé celui ci d'un trou permettant également l' introduction de la prothèse artificielle. A cette même extrémité, des bagues et rondelles (11,12) en acier inoxydable ou en matière plastique biocompatible, permettent de maintenir en place une valve élastique (14 en position fermée et 14 bis en position ouverte ) qui peut s'ouvrir sous la poussée d'une aiguille de remplissage ou d'une sonde d'intervention, puis se refermer pour obturer le réservoir après extraction de l'aiguille ou de la sonde, elle s'oppose lors du remplissage du Réservoir Principal en produit actif ou lors de l'enfilage d'une sonde d'exploration ou de curage, à la fuite de ce produit actif ou de sang. Une vis (13) vissée dans la rondelle (12) complète l'étanchéité de l'ensemble.

Les pièces (11,12) sont reliées entre elles et à la paroi (1) de façon étanche soit par vissage, collage, sertissage, soudure ou toute autre méthode appropriée., un produit (15) facilitant la liaison et l'ossification entre la paroi (1) et l'os du sternum, par exemple une poudre de corail biologique, peut être incorporée lors de la fabrication de la prothèse à la surface de cette paroi (1) pour faciliter l'ancrage définitif au sternum

Dans l'application où la prothèse artificielle est destinée à Remplacer purement et simplement une portion de vaisseau défaillant une tubulure externe (16) en position dégonflée, (16 bis) en position de remplissage maximum, réalisée dans un matériau élastique, prolonge la paroi (1,1bis) du " Réservoir Principal ", et une tubulure interne concentrique (17), sert de dérivation à la circulation sanguine pour court-circuiter la partie de vaisseau défaillante, un espace vide (18) sépare ces deux parois concentriques.

La tubulure interne (17) est micro-poreuse ou percée de nombreux micro-trous (19) de très faible diamètre permettant une diffusion très lente des produits actifs contenus en (2) dans le ou les réservoirs principaux et dans le vide (18), des pièces de raccordement (4) assurent la liaison en amont et en aval de la dérivation entre la tubulure (17) et le vaisseaurécepteur, l'anastomose entre prothèse et vaisseau récepteur se fait comme précédemment par l'intermédiaire des plaques courbes (4bis).

Pour pouvoir traverser la Pastille micro-poreuse amovible (6) on la paroi de la tubulure interne (17) de la prothèse il doit régner dans le ou les réservoirs une pression légèrement supérieure à celle de la pression sanguine, c'est pourquoi la paroi externe (16) de la prothèse en dérivation et celle du on des " Réservoirs Principaux "(1,1 bis) la prolongeant est prévue dilatable et élastique, les remplissages de l'ensemble prothèse/réservoirs se font donc sous une légère pression qui diminue lentement an fur et à mesure de la vidange par diffusion afin de rester toujours légèrement plus forte que le pression sanguine moyenne jusqu'au prochain remplissage. L'élasticité de ces parois est la source de la force qui pousse le liquide actif au travers des Pastilles micro-poreuses amovibles (6) ou de la paroi interne (17) de la prothèse en dérivation, cette élasticité est elle même réglable en fonction de la nature du matériau biocompatible constituant les parois externes, par exemple un silicone, un latex anallergique, un polyuréthane, un polymère etc.. et de leur épaisseur.

Pour éviter que la tubulure (17) ne s'aplatisse sous cette légère surpression un ressort (20), genre ressort à boudin, généralement réalisé en acier inoxydable biocompatible, peut être incorpore à la fabrication dans l'épaisseur de la paroi de la tubulure interne (17)

L'extrémité aval du Réservoir Principal est légèrement différente: la pastille (6) est supprimée et remplacée par une bague (21) percée de quelques petits trous (22) permettant au produit actif de passer librement du Réservoir Principal à l'espace (18), en outre une bague (23) reçoit une vis ou un bouchon (24) qui sont destinés à empêcher toute communication entre le Réservoir Principal (1,1bis) et la tubulure interne (17), le liquide actif ne peut ainsi pénétrer à l'intérieur de la tubulure (17) qu' après avoir traversé la paroi micro-poreuse ou percée de micro-trous (17). Une bague (25) sertie autour des éléments (21,23,24) assure la liaison étanche de ces éléments entre eux.

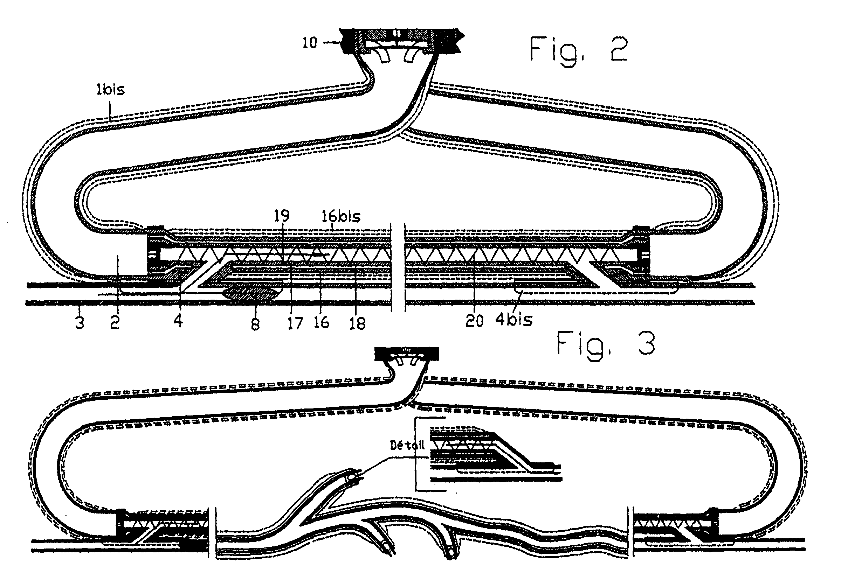

La dérivation ainsi réalisée peut ne comporter qu'un seul Réservoir Principal à son extrémité amont ou en comporter deux l'un à l'amont l'autre à l'aval de la dérivation comme dessiné Fig.2

Si deux réservoirs sont prévus leurs extrémités amont peuvent se réunir et se fondre l'une dans l'autre ou au contraire être munis chacun d'un même ensemble d'éléments (10 à 15) et venir s'insérer côte à côte ou l'un sous l'autre par exemple dans le sternum.

La tubulure (17) est réalisée dans un matériau biocompatible dont de nombreux exemples sont donnés dans de multiples publications et en particulier dans le brevet N° FR 2785812, il peut s'agir de métaux, de polymères, de matériaux composites, d'élastomères, de céramiques et de fibres de carbone, certains d'entre eux peuvent être traités afin d'avoir une porosité convenable, il est également possible d' incorporer dans la paroi du conduit (17) des nano-tubes en carbone en quantité calculée de façon à obtenir la porosité souhaitée.

Si la paroi du conduit (17) n'est pas micro-poreuse elle sera percée de micro-trous (19) de quelques dizaines de nanomètres à quelques dizaines de micromètres de diamètre.

Les tubulures (1 et 16) seront fabriquées dans des matériaux possédant des propriétés élastiques tels le silicone, le latex anallergique, divers polymères, entre autres.

Ces conduits seront fabriqués, en une ou plusieurs pièces, à l'aide de techniques connues propres à chacun des matériaux utilisés.

La pièce de raccordement (4), également en matériau biocompatibe et formant un tout avec la plaque courbe (4bis), sera soit moulée ou usinée en même temps que les tubulures (1 et 16), soit plus commodément moulée ou usinée séparément puis collée ou soudée (par ultra-sons par exemple). Le rayon de courbure de la plaque (4bis) sera adapté au rayon des vaisseaux sur lesquels elle est raccordée.

## Revendications

1. Prothèse vasculaire artificielle bio active
**caractérisée en ce qu'**elle comprend :
- un ou des réservoirs de produits actifs de grande capacité (2) et à parois élastiques (1, 1bis),
- une extrémité du ou des réservoirs étant facilement accessible à tout moment sans nécessiter une intervention chirurgicale et étant équipée d'une valve (14,14 bis) et d'une vis on bouchon (13) empêchant toute fuite pendant et après le remplissage, sous légère pression, du on des réservoirs en produits actifs à l'aide, par exemple, d'une aiguille hypodermique, et/ou pendant l'introduction de divers instruments et sondes destinés à l'exploration et au débouchage des prothèses vasculaires et des vaisseaux naturels,
- une extrémité opposée du ou des réservoirs équipée d'une pièce de raccordement (4) assurant une importante surface courbe de contact facilitant le collage provisoire et/ou définitif de la prothèse avec les vaisseaux et/ou les organes naturels (3), la périphérie de la pièce de raccordement (4) pouvant être percée d'un grand nombre de trous (5) destinés à être comblés par des cellules proliférantes dans le but d'assurer une liaison et une étanchéité particulièrement efficaces,
la dite prothèse permettant la délivrance en permanence et si besoin pendant toute la vie des patients de produits biologiquement actifs dans un ou plusieurs vaisseaux et/ou organes récepteurs notamment des médicaments délivrés In Situ pour le traitement de divers organes et maladies ainsi que des produits capables de s'opposer à la prolifération de tissus indésirables et à la formation de bouchons.

2. Prothèse artificielle bio active selon la revendication 1 **caractérisée en ce que** l'extrémité aval du réservoir (2,1,1bis) sous légère pression reliée au vaisseau et/ou à l'organe naturel (3) est munie d'une pastille (6) à faible perméabilité permettant un passage permanent et contrôlé de médicaments et/ou de produits actifs dans le ou les organes à traiter et/ou les vaisseaux naturels et dans le sang.

3. Prothèse artificielle bio active selon la revendication 1 destinée à servir de dérivation doublant et remplaçant des parties de vaisseaux devenues totalement non fonctionnelles, **caractérisée en ce qu'**elle comprend deux conduits concentriques distincts (16,16bis,17) de longueur sensiblement égale ou supérieure à celle des segments de vaisseaux dérivés; le conduit externe (16,16bis) est le prolongement des parois (1,1bis) du réservoir et est réalisé dans un matériau élastique similaire; le conduit interne (17), renforcé si besoin par un ressort à boudin (20), est constitué d'un matériau très légèrement poreux ou percé de micro-trous (19) permettant une diffusion extrêmement lente des produits actifs; ses extrémités sont reliées au vaisseau naturel (3) à l'amont et à l'aval de la dérivation par des pièces de raccordement (4); et elle comporte en outre un système (21 à 25) permettant l'introduction d' instruments permettant l'exploration et/on le débouchage de la prothèse et du ou des vaisseaux de l'amont vers l'aval mais empêchant toute communication entre l'intérieur du conduit interne (17) et le réservoir (1,1bis) par un bouchon ou une vis (24) d'obturation manoeuvrables, grâce à un outil flexible, depuis l'entrée de remplissage du réservoir, et par une bague (23) percée de quelques trous (22) permettant le passage des produits actifs du réservoir à l'espace vide (18) existant entre les deux conduits concentriques (16,17).

4. Prothèse artificielle bio active selon la revendication 3 **caractérisée en ce qu'**elle comprend un deuxième réservoir, similaire au précédent, rajouté à l'extrémité aval du conduit externe en dérivation doublant ainsi le volume de produits actifs stockés et permettant également l'introduction d'instruments permettant l'exploration et/ou le débouchage de la prothèse et du vaisseau naturel de l'aval vers l'amont.

5. Prothèse artificielle bio active selon l'une des revendications 1 à 3 **caractérisée en ce que** elle comprend des produits (15), facilitant la liaison solide et durable entre prothèses et vaisseau et/ou organes, incorporés lors de la fabrication et disposés à l'extérieur de l'extrémité amont de la paroi (1,1bis) du réservoir reliée au sternum (10) ainsi qu'à l'intérieur des surfaces courbes des pièces de raccordement (4).

## Revendications modifiées

### Revendications modifiées conformément à la règle 86(2) CBE.

**1.** Prothèse vasculaire artificielle bio active comprenant
- un ou des réservoirs de produits actifs à parois élastiques,
- une extrémité du ou des réservoirs étant facilement accessible à tout moment sans nécessiter une intervention chirurgicale et une extrémité opposée du ou des réservoirs équipée d' une pièce de raccordement assurant une importante surface courbe de contact, facilitant le collage provisoire et/ou définitif de la prothèse avec les vaisseaux et/ou les organes naturels, la dite prothèse permettant la délivrance en permanence et si besoin pendant toute la vie des patients de produits biologiquement actifs dans un ou plusieurs vaisseaux et/ou organes récepteurs notamment des médicaments délivrés In Situ pour le traitement de divers organes et maladies ainsi que des produits capables de s'opposer à la prolifération de tissus indésirables et à la formation de bouchons.
**caractérisée en ce que** :
- ces prothèses vasculaires artificielles sont aussi bien reliables directement à un vaisseau ou un organe déficient que posées en dérivation pour ponter une partie de vaisseau ou d'organe défaillant, cette double potentialité permet donc aussi bien un apport direct de médicaments et de produits actifs dans le sang, dans un vaisseau malade ou défaillant, dans un organe malade afin de le ou de les traiter, sans limitation dans le temps, que le remplacement pur et simple d'un vaisseau défaillant,
- la très grande capacité des réservoirs (2,18) pour produits actifs fait qu'il n'est nécessaire de les recharger qu'à intervalles de temps très éloignés,
-- les parois (1,1bis) des réservoirs (2) sont élastiques pour générer une force de poussée capable de propulser les produits actifs au travers des pastilles (6) et des parois poreuses ou percées de trous (17),
- des pastilles (6) poreuses ou percées de nombreux trous de très faible diamètre permettent de réguler les quantités de produits actifs délivrées,
- des parois doubles concentriques (16,16bis,17) peuvent prolonger ces réservoirs (2) jusqu'à une ou plusieurs pièces de raccordement (4) avec les vaisseaux ou organes à desservir, la paroi interne (17) est poreuse ou percée d'un grand nombre de trous de très faible diamètre pour permettre le passage contrôlé des produits actifs, la paroi (16,16bis) est élastique dans le même but que les parois (1,1bis), l'espace (18) séparant ces deux parois sert de réservoir complémentaire,
- les réservoirs (2) sont équipés d'une valve (14,14 bis) maintenus en place par des rondelles (11,12) et d'une vis ou bouchon (13) empêchant toute fuite pendant et après le remplissage, sous légère pression, du ou des réservoirs en produits actifs à l'aide, par exemple, d'une aiguille hypodermique, et/ou pendant l'introduction de divers instruments et sondes destinés à l'exploration et au débouchage des prothèses vasculaires et des vaisseaux naturels,
- le diamètre de la partie courbe des pièces de raccordement (4) est choisi plus ou moins grand pour s'adapter à la taille des vaisseaux à raccorder,
- la périphérie des pièces de raccordement (4) peut être percée d'un grand nombre de trous (5) destinés à être comblés par des cellules proliférantes dans le but d'assurer une liaison et une étanchéité particulièrement efficaces, la qualité des collages provisoires et/ou définitifs permise par l'importance des surfaces en contact permet de se passer dans de nombreux cas des anastomoses classiques par fil et aiguille,
- la propulsion des produits actifs au travers de la pastille (6) et ou de la paroi (17) ne nécessite aucun mécanisme ni aucune énergie d'apport car elle est assurée par l'élasticité des parois (1,1bis,16,16bis), mettant cette prothèse à l'abri de tout problème d'apport d'énergie et d'autonomie.

**2.** Prothèse artificielle bio active selon la revendication 1 **caractérisée en ce que** l'extrémité aval du réservoir (2,1,1bis) sous légère pression reliée au vaisseau et/ou à l'organe naturel (3) est munie d'une pastille (6) à faible perméabilité permettant un passage permanent et contrôlé de médicaments et/ou de produits actifs dans le ou les organes à traiter et/ou les vaisseaux naturels et dans le sang.

**3.** Prothèse artificielle bio active selon la revendication 1 destinée à servir de dérivation doublant et remplaçant des parties de vaisseaux devenues totalement non fonctionnelles, **caractérisée en ce qu'**elle comprend deux conduits concentriques distincts (16,16bis,17) de longueur sensiblement égale ou supérieure à celle des segments de vaisseaux dérivés; le conduit externe (16,16bis) est le prolongement des parois (1,1bis) du réservoir et est réalisé dans un matériau élastique similaire; le conduit interne (17), renforcé si besoin par un ressort à boudin (20), est constitué d'un matériau très légèrement poreux ou percé de micro-trous (19) permettant une diffusion extrêmement lente des produits actifs; ses extrémités sont reliées au vaisseau naturel (3) à l'amont et à l'aval de la dérivation par des pièces de raccordement (4); et elle comporte en outre un système (21 à 25) permettant l' introduction d' instruments permettant l'exploration et/ou le débouchage de la prothèse et du ou des vaisseaux de l'amont vers l'aval mais empêchant toute communication entre l'intérieur du conduit interne (17) et le réservoir (2,1,1bis) par un bouchon ou une vis (24) d'obturation manoeuvrables, grâce à un outil flexible, depuis l'entrée de remplissage du réservoir, et par une bague (23) percée de quelques trous (22) permettant le passage des produits actifs du réservoir (2) à l'espace vide (18) existant entre les deux conduits concentriques (16,16bis,17) dans ce cas la pastille (6) peut être supprimée.

**4.** Prothèse artificielle bio active selon la revendication 3 **caractérisée en ce qu'**elle comprend un deuxième réservoir, similaire au précédent, rajouté à l'extrémité aval du conduit externe (16) en dérivation doublant ainsi le volume de produits actifs stockés et permettant également l' introduction d' instruments permettant l'exploration et/ou le débouchage de la prothèse et du vaisseau naturel de l'aval vers l'amont.

**5.** Prothèse artificielle bio active selon l'une des revendications 1 à 4 **caractérisée en ce que** elle comprend des produits (15), facilitant la liaison solide et durable entre prothèses et vaisseaux et/ou organes, incorporés lors de la fabrication et disposés à l'extérieur de l'extrémité amont de la paroi (1,1 bis) du ou des réservoir reliée au sternum (10) ainsi qu'à l'intérieur des surfaces courbes des pièces de raccordement (4).

**6.** Prothèse artificielle bio active selon revendications 1 à 5 **caractérisée en ce qu'**elle est de dimensions suffisamment petites pour être mise en place non seulement par voie de chirurgie classique mais également par voies de chirurgie endoscopique et /ou mini-invasive
